# EUROPEAN PATENT SPECIFICATION

(11) **EP 2 178 479 B1**
(45) Date of publication and mention of the grant of the patent: **17.06.2015**
(21) Application number: 08782159.1
(22) Date of filing: 21.07.2008
(51) Int. Cl.: H04R 25/00, A61C 8/00

(54) **COUPLING APPARATUS FOR A BONE ANCHORED HEARING DEVICE**
KUPPLUNGSGERÄT FÜR EIN IN KNOCHEN VERANKERTES HÖRGERÄT
APPAREIL D'ACCOUPLEMENT POUR UN DISPOSITIF AUDITIF ANCRÉ SUR UN OS

(30) Priority: 20.07.2007 US 951169 P; 20.07.2007 US 951163 P
(43) Date of publication of application: 28.04.2010
(73) Proprietor: Cochlear Americas, Englewood, CO 80112 (US)
(72) Inventor: JINTON, Lars, Lane Cove, NSW 2066 (AU); HOLGERSSON, Erik, Lane Cove, NSW 2066 (AU); ELMBERG, Peter, Lane Cove, NSW 2066 (AU)
(74) Representative: Grünecker Patent- und Rechtsanwälte PartG mbB
(86) International application number: PCT/US2008/070681
(87) International publication number: WO 2009/015103

(56) References cited:
- EP-B1- 0 996 391
- WO-A1-02/09622
- WO-A1-96/19950
- WO-A1-2004/045432
- US-A- 4 936 317
- US-A- 5 833 463
- US-A1- 2006 172 257
- US-A1- 2007 009 853
- US-A1- 2007 059 666
- US-B2- 6 669 701
- US-B2- 7 065 223
- SJOSTROM M ET AL: "Monitoring of implant stability in grafted bone using resonance frequency analysis - A clinical study from implant placement to 6 months of loading", INTERNATIONAL JOURNAL OF ORAL AND MAXILLOFACIAL SURGERY, COPENHAGEN, DK, vol. 34, no. 1, 1 January 2005 (2005-01-01), pages 45-51, XP004691019, ISSN: 0901-5027

## Description

### CROSS-REFERENCE TO RELATED APPLICATIONS

This application claims the benefit of U.S. Provisional Application No. 60/951,169, entitled "Coupling Apparatus for a Bone Anchored Hearing Device," filed July 20, 2007 and U.S. Provisional Application No. 60/951,163, entitled "Bone Anchor Fixture for a Medical Prosthesis," filed July 20, 2007.

### BACKGROUND

### Field of the Invention

The present invention relates generally to hearing devices and, more particularly, to a coupling apparatus for bone anchored hearing devices.

### Related Art

For persons who cannot benefit from traditional, air conduction hearing aids there are other types of hearing aids on the market commonly referred to as bone anchored hearing aids. Bone anchored hearing aids mechanically transmit sound information to a person's inner ear via the skull bone by means of a vibrator. Such hearing aid devices are typically connected to a percutaneous implant in the form of a titanium screw implanted in the skull bone behind the external ear so that sound is transmitted via the skull bone to the cochlea (inner ear). This enables the hearing aid to be effective regardless of whether there is disease or damage in the middle ear. Moreover, penetration of the skin makes the vibratory transmission very efficient.

Bone anchored hearing aids were initially developed to rehabilitate certain types of hearing-impaired patients. They may also be utilized for other indications such as stuttering and for certain non-medical applications. A bone anchored hearing aid may be connected to an implant by means of a bayonet coupling, a snap-in coupling, a magnetic coupling or the like. One example of this type of hearing aid device is the BAHA® bone anchored hearing aid, described in U.S. Patent No. 4,498,461 and commercially available from Cochlear Bone Anchored Solutions AB (previously Entific Medical Systems AB) in Göteborg, Sweden.

Typically, the implant connecting the hearing aid to the skull generally comprises two components: a bone attachment piece that is attached or implanted directly into the skull bone and a skin penetrating piece coupled to the bone attachment piece. This two-piece design facilitates the installation of the implant in two sequential steps. In the first step, the bone attachment piece is installed into the skull and the surrounding tissue is allowed to heal for a period of time that may last up to a few months. In the second step, the skin penetrating piece is coupled to the bone attachment piece. In the event that the skin penetrating piece becomes damaged, it may be replaced without removing the anchoring fixture from the skull. Moreover, this permits the hearing aid to be changed or upgraded, if necessary, without removing the bone attachment piece from the skull.

A well-known problem with many of these conventional percutaneous implants is the increased risk of infections and inflammation that results from bacterial colonization in the area surrounding the skin-implant interface. Oftentimes, unwanted pockets and gaps are formed between the skin and the implant. In conventional implants such gaps are dimensioned so as to provide an ideal environment for bacterial growth, inflammation and infection in the tissue surrounding the implant.

Another problem with these conventional percutaneous implants is the risk of micro-leakage and bacterial colonization from gaps formed between the two components. Such gaps result from insufficient connection between bone attachment piece and the skin penetrating piece, imperfections in the contact surfaces of the two components, incorrect tightening torque, or a combination of the foregoing factors.

A coupling apparatus for anchorage of a prosthesis to a patient's skull bone is known from US 7 065 223 B2 or US 2007/009853 A1 showing a hearing aid bone anchor. US 2007/059666 A1, US 2006/172257 A1, and WO 96/19950 A1 show a similar coupling apparatus linked to a dental bone anchor. US 7 065 223 B2 discloses the features of a coupling apparatus according to the preamble of claim 1. 2

### SUMMARY

In order to overcome the above problem the present invention provides a coupling apparatus according to claim 1. Further embodiments are defined in the dependent claims. In one embodiment, a coupling apparatus for anchorage of a prosthesis to a patient's skull bone is disclosed. The coupling apparatus comprises an elongate anchoring fixture comprising an external screw thread for penetrating the bone, and a flange that abuts the bone when the fixture is implanted. The fixture has a proximate end having an open cavity with a tapered interior contour. The skin-penetrating abutment is detachably connectable to the fixture and comprises a substantially conical exterior surface with a wider proximate region and a narrower distal region having a tapered exterior contour that fits within said tapered interior contour of the open cavity.

In another embodiment, a coupling apparatus for a bone anchored prosthesis is disclosed. The coupling apparatus comprises a bone anchor fixture and an abutment sleeve coupled to the bone anchor fixture. The bone anchor fixture comprising an outer screw thread and a flange. The flange has a flared outer top surface and a bottom portion configured to prevent the bone anchor fixture from penetrating through the bone. The abutment sleeve is coupled to the bone anchor fixture and comprises a tapered outer surface adjacent to the flared outer top surface of the flange. The flared outer top surface of the bone anchor fixture and the tapered outer surface of the abutment sleeve define an outer contour having a substantially hourglass shape having a waist and a waist angle.

In yet another embodiment, a coupling apparatus for a bone anchored prosthesis is disclosed. The coupling apparatus comprises a bone anchor fixture comprising an outer screw thread and a flange having an open cavity and tapered inner side walls disposed within the open cavity. The coupling apparatus further comprises an abutment sleeve comprising a tapered outer surface configured to be seated within the tapered inner side walls of the bone anchor fixture.

In a further embodiment, a healing cap for use with a coupling apparatus for a bone anchored prosthesis is disclosed. The healing cap comprises a platform having a central opening and one or more protruding members configured to removably attach the healing cap to coupling apparatus. A cap is provided for the central opening of the platform and a flexible band connects the platform and the cap.

### BRIEF DESCRIPTION OF THE DRAWINGS

Embodiments of the present invention are described herein with reference to the attached drawing sheets in which:
FIG. 1 is a perspective view of the coupling apparatus in accordance with one embodiment of the coupling apparatus;
[FIG. 2 is a side view of the embodiment of the coupling apparatus illustrated in FIG. 1;
FIG. 3 is a cross-sectional side view of the embodiment of the coupling apparatus illustrated in FIG. 1;
FIG. 4 is a partial view of a proximate end of the fixture illustrating the tapered connection between the fixture and the abutment components of the coupling apparatus illustrated in FIG. 1, in accordance with one embodiment of the coupling apparatus;
FIG. 5A is a perspective view of one embodiment of an elongate coupling shaft having a screw head with an external hex and a bore with an internal screw thread;
FIG. 5B is a perspective view of another embodiment of an elongate coupling shaft having a screw head with an external hex and a bore with an internal screw thread;
FIG. 6A is a side view of a healing cap with the coupling apparatus illustrated in FIG. 1, with the healing cap in a closed orientation;
FIG. 6B is a perspective view of the healing cap illustrated in FIG. 6A;
FIG. 7A is a side view of the healing cap illustrated in FIGS. 6A and 6B, with the healing cap in an open orientation; and
FIG. 7B is a perspective view of the healing cap illustrated in FIG. 7A.

### DETAILED DESCRIPTION

Aspects and exemplary embodiments of the invention disclosed herein are generally directed to a bone anchored coupling apparatus in which the surfaces of an implanted fixture component and an associated abutment component are configured so as to reduce gaps thereby minimizing undesirable micro-leakage, preferably regardless of any imperfections in the mating surfaces and a range of tightening torques.

In certain embodiments, the coupling apparatus has a smooth outer contour facing the surrounding soft tissue to minimize unwanted pockets or gaps in the tissue-device interface. In particular embodiments, the regions of the fixture and the abutment components which mate with each other have inverse conically-shaped regions such that, when mated, the fixture forms the bottom of an hourglass shape while the abutment forms the top of the hourglass shape. Preferably, the dimensions of the hourglass configuration are such that the patient's skin abuts the narrow region of the hourglass configuration.

In certain embodiments, the upper end surface of the fixture has an open cavity with a tapered interior surface forming a seat for the tapered exterior side wall of the abutment. This preferably creates a good connecting fit between the fixture and abutment so as to reduce the risk of micro-leakage.

A well known problem with percutaneous implants is infection and inflammation at the skin-implant interface resulting from bacterial colonization in the area around the interface. The coupling apparatus disclosed herein increases the integration of the skin to the coupling apparatus thereby decreasing the likelihood that a gap will form between the two. Such gaps are, unfortunately, an ideal environment for the bacteria. By creating an integration of the skin to the coupling apparatus the adverse skin reactions associated with bone anchored percutaneous implants may be reduced.

Integration between the skin and the implant occurs when the soft tissue encapsulates the implant in fibrous tissue and does not readily dissociate itself from the implant. U.S. Provisional Patent Application No. 60/951,163, entitled "Bone Anchor Fixture for a Medical Prosthesis", filed July 20, 2007, discloses a surface modification which reduces certain adverse skin reactions, and which may be implemented in embodiments of the present invention. In other embodiments the abutment sleeve is coated to reduce the shear modulus, as described in greater detail below.

Embodiments of the coupling apparatus may be used in connection with hearing aid devices of the bone conduction type, that is, hearing aid devices by which the sound is transmitted via the skull bone directly to the inner ear of a person with impaired hearing. However, embodiments of the coupling apparatus may also be configured for use in connection with other types of hearing aid devices anchored in the skull bone and for ear or orbital prostheses which are also anchored in the skull bone. Other applications of the coupling apparatus are also contemplated.

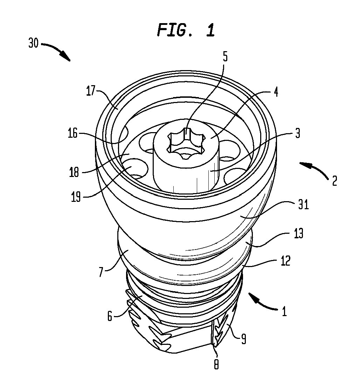

Embodiments of the bone anchored coupling apparatus will be described below with reference to the accompanying drawings. FIG. 1 is a perspective view of a bone anchored coupling apparatus 30 according to one embodiment. Coupling apparatus 30 is illustrated in FIG. 1 with a screw-shaped bone anchor fixture 1 and a skin-penetrating abutment sleeve 2. The two components are connected by an elongate coupling shaft or an abutment screw 3 having, in one embodiment, a cylindrical screw head 4. Screw head 4 has an internal hex or multi-lobular configuration 5 for a cooperating insertion tool, which is not part of the invention and therefore not illustrated here.

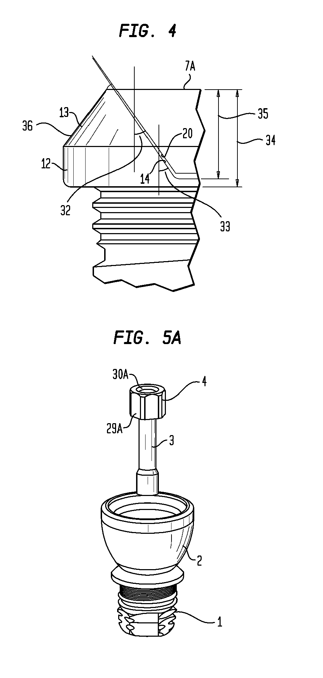

In an alternate embodiment depicted in FIG. 5A, a coupling shaft 3 is illustrated as having a screw head 4 with an external hex geometry 29A and a bore with an internal screw thread 30. The external hex geometry 29A may be used for the screw tightening and the internal screw thread 30 may be used for connecting measuring probes or the like for controlling purposes, such as a Resonance Frequency Analyzer (RFA) probe for measuring implant stability and osseointegration. In one embodiment, an M 1.6 screw is used. In combination with the conical fit between the fixture 1 and the abutment sleeve 2, the M 1.6 screw provides sufficient torque to secure the assembly and improved elastic deformation of the screw. This reduces the likelihood of the coupling assembly 30 become disassembled or unscrewed.

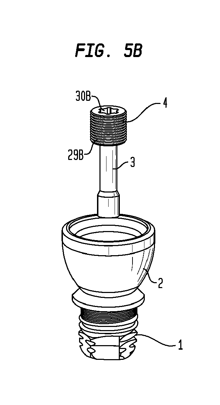

In the alternative embodiment illustrated in FIG. 5B, rather than external hex geometry 29A, screw head 4 has outer threads 29B and an internal unigrip 30B. Such an embodiment may be suitable for connecting certain RFA probes.

Fixture 1 may be made of any material that has a known ability to integrate into surrounding bone tissue, a phenomenon commonly referred to as osseointegration. In one embodiment, fixture 1 is made of titanium. Fixture 1 has a main body with an outer screw thread 6 which is intended to be installed into the skull bone. Fixture 1 also comprises a flange 7 configured to function as a stop when fixture 1 is installed into the skull bone. Flange 7 prevents the screw thread 6 from completely penetrating through the skull bone. Fixture 1 may further comprise a tool-engaging socket having an internal grip section (now shown) for easy lifting and handling of fixture 1. Tool-engaging socket and the internal grip section are described and illustrated in co-pending U.S. Provisional Application No. 60/951,163, entitled "Bone Anchor Fixture for a Medical Prosthesis," filed July 20, 2007.

The main body of fixture 1 has a length sufficient to securely anchor the fixture 1 into, without penetrating entirely through, the skull bone. The length of the main body may therefore depend on the thickness of the skull bone at the implantation site. In one embodiment, the main body has a length that is no greater than 5 mm, measured from the planar bottom surface 10 of the flange 7 to the end of the distal region 1B. In another embodiment, the length of the main body is from about 3.0 mm to about 5.0 mm.

In the embodiment depicted in FIGS. 1-3, the main body has a tapered apical proximate end 1A, a straight, generally cylindrical body, and a screw thread 6. The distal region 1B of fixture 1 is fitted with three self-tapping cutting edges 8 formed into the exterior surface of the fixture. Further details of the self-tapping features are described in International Patent Application WO 02/09622.

A clearance or relief surface 9 may be provided adjacent to the self-tapping cutting edges 8. This design is advantageous because it reduces the squeezing effect between the fixture 1 and the bone during installation of the screw by creating more volume for the cut-off bone chips.

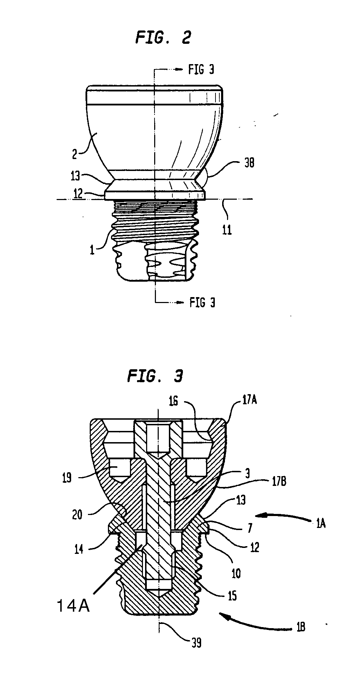

As more clearly illustrated in FIGS. 2 and 3, flange 7 has a planar bottom surface 10 for resting against the outer bone surface, indicated by 11 in FIG. 2, when anchoring fixture 1 has been screwed down into the skull bone. Again, flange 7 prevents the fixture from completely penetrating through the skull bone. Preferably, flange 7 has a diameter which exceeds the peak diameter of the screw threads 6. In one embodiment, the diameter of the flange 7 exceeds the peak diameter of the screw threads 6 by approximately 10-20%. Although flange 7 is illustrated in FIGS. 1-5 as being circumferential, flange 7 may be configured in a variety of shapes so long as flange 7 has a diameter or width that is greater than the peak diameter of the screw threads 6. Also, the size of flange 7 may vary depending on the particular application for which the coupling apparatus 30 is intended.

The outer peripheral surface 36 of flange 7 has a cylindrical part 12 and a flared top portion 13. Upper end 7A of flange 7 is designed with an open cavity 14A having a tapered inner side wall 14. The tapered inner side wall 14 is adjacent to the grip section (not shown). The interior of the flange 7 further includes an inner bottom bore 15 having internal screw threads for securing a coupling shaft 3.

In one embodiment, increased stability to the coupling between fixture 1 and abutment sleeve 2 is provided by having the length of the open cavity 14A extends from flange 7 to the distal region 1B of fixture 1. Tapered inner side wall 14 forms a seat for abutment sleeve 2 to create a good connecting fit between the fixture 1 and abutment sleeve 2. As shown in FIG. 4, a cone angle 33 is defined by the tapered interior contour 14 relative to a vertical axis. In one embodiment, the cone angle 33 may be in the range of approximately 30-40 degrees.

In the embodiments illustrated in the figures, the flange 7 has a smooth, open upper end 7A and does not have a protruding hex. The smooth upper end 7A of the flange 7 and the absence of any sharp corners provides for improved soft tissue adaptation. Flange 7 also comprises a cylindrical part 12 which, together with the flared upper part 13, provides sufficient height 34 in the longitudinal direction for internal connection with the abutment sleeve 2. FIG. 4 illustrates the tapered seat 14 seated within the height 34 of flange 7.

The skin penetrating part of the implant includes the abutment sleeve 2. In one embodiment, abutment sleeve 2 has an inner annular flange 16 at its upper edge 17A configured to cooperate with other components (not shown) by means of snap-in connection or the like. Abutment sleeve 2 has an internal shoulder 18 with a central opening for a connecting shaft or coupling shaft 3 and a number of peripherally arranged through holes or recesses 19 used for a tool such as that illustrated in WO 2004/105650.

It should be appreciated that the fixture 1, abutment sleeve 2 and coupling shaft 3, may be delivered separately or they may be delivered in the form of a pre-mounted device as illustrated in WO 2004/105650. In accordance with one embodiment, the coupling apparatus 30 is delivered to the surgeon pre-mounted in its package to facilitate installation of the entire device in a single step. Abutment sleeve 2 may be pre-mounted to the fixture 1 at the manufacturing site with the correct tightening torque to obviate the need for the surgeon to know the correct tightening torque or to handle the separate pieces of the coupling apparatus 30.

In contrast to traditional implants which require an outer fixture hex for tool engagement, the coupling apparatus 30 may be installed by using the recesses 19 in the abutment sleeve 2. These recesses 19 are located on the upper part of the coupling apparatus 30 and are more visible than a traditional outer hex.

According to the embodiment shown in FIG. 3, the abutment sleeve 2 has a substantially curved, conical outer surface with an upper edge 17A and a bottom, fixture-connecting part 17B. The upper edge 17A may have a width or diameter that is larger than that of the bottom, fixture connecting part 17B. The bottom part 17B of the outer surface has a contour 20 adapted to be seated with the tapered inner contour 14 of the fixture to create a good connecting fit between the fixture 1 and abutment sleeve 2. The two conical shaped surfaces not only provides an axially well-defined fit when assembled together, they also provides for easy disassembly.

It would be desirable to reduce the risk for micro-leakage from the coupling apparatus 30. Designing the upper part of the fixture 1 and the lower part of the abutment sleeve 2 with a conical fit reduces the risk for gaps and unwanted micro-leakage, regardless of any imperfections in the contact surfaces or incorrect tightening torques.

As shown in FIG. 4, the tapered portion 20 of the abutment sleeve 2 is characterized by a cone angle 32. The cone angle 32 is configured so as to securely couple the fixture 1 and the abutment sleeve 2 without significant gaps. Preferably, the cone angles 32 and 33 are in the range of about 30° to about 40°, with little or no difference between the cone angles 32 and 33. In one embodiment, the cone angles 32 and 33 are substantially the same. In another embodiment, the difference between the cone angles 32 and 33 is about 1° to about 5°, and more preferably about 1°.

It would also be desirable to reduce the risk of infections and inflammation that results from bacterial colonization in the unwanted pockets and gaps that are formed between the skin and the implant. As illustrated in FIG. 2, an hourglass waist angle 38 is generally defined between the exterior peripheral surface 36 of flared portion 13 of flange 7 and the tapered lower portion 20 of abutment sleeve 2. The hourglass waist angle 38 provides a smooth outer contour to the facing soft tissue such that unwanted pockets and gaps are not formed between the surrounding tissue and the coupling apparatus 30. This smooth contour facilitates integration between the coupling apparatus 30 and the surrounding tissue to substantially eliminate gaps and unwanted pockets where bacteria might grow. This is in contrast to many traditional implant devices in which a comparatively sharp interface is formed with the contacting tissue. The embodiment depicted in FIG. 2 show an hourglass waist angle is greater than 90°.

In certain embodiments, the skin-contacting surface of abutment sleeve 2 may be modified in such a way that the shear modulus of the skin-contacting part of abutment sleeve 2 is reduced to less than 35 GPa. The surface of the skin-contacting part of the percutaneous implant abutment sleeve 2 may be coated with a biocompatible polymer or a ceramic material. In accordance with one aspect of the embodiments, the coating has a thickness of approximately 0.001-50.0 µm. A surface increasing treatment may be provided resulting in a roughness value Sₐ of 0.5-10 µm in place of or in addition to the coating. These modifications to the skin-contact surface generally reduce the shear modulus and certain adverse skin reactions. Surface modifications of this type are discussed in more detail in the co-pending patent application.

In certain embodiments, the abutment sleeve is coated with a material to reduce the shear modulus. Such a material may be, for example, a biocompatible polymer, a ceramic material, and/or a combination thereof. In one specific embodiment, the material has a thickness of about 0.001 µm to about 50.0 µm. In the same or other embodiments, the shear modulus is reduced to less than 35 GPa. In the same or other embodiments, the abutment sleeve is coated with a surface increasing material. Such a material may be such that it provides a roughness value of about 0.5 µm to about 10 µm. Further details of the above and other features may be found in Swedish Patent Application No. 0701 244-6, also published as SE 531 177 C2.

The coupling apparatus 30 may also be designed in such a way that it is easy to handle together with instruments and components used for installation and control of the implant device. As noted, surgical techniques normally used for installing the implants have been carried out as a two-step procedure. In the first step the implant is inserted and maintained unloaded during a healing period of about a couple of months. During this healing period it is important to avoid micro-leakage from the coupling apparatus and bacteria colonization.

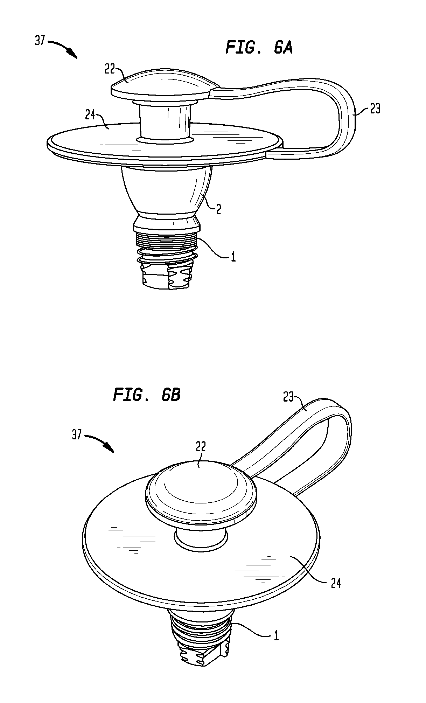

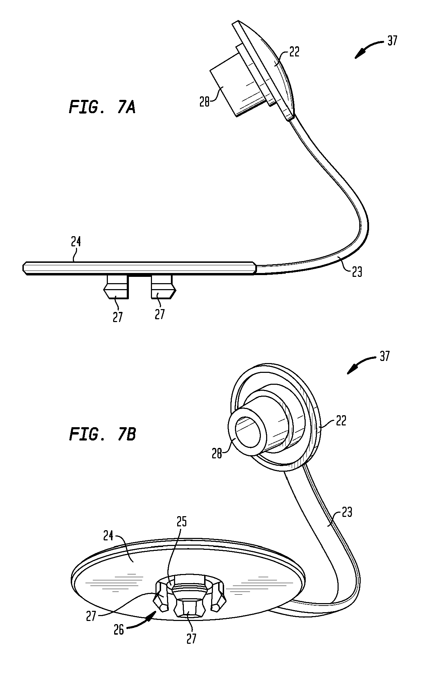

FIGS. 6-7 depict a healing cap 37 that may be used in connection with the coupling apparatus 30. As illustrated in FIGS. 6-7, the healing cap 37 comprises a snap-in platform 24, a cap 22, and a flexible band 23 connecting the snap-in platform 24 and the cap 22. This design makes it easy and safe for the surgeon to handle.

Snap-in platform 24 includes a central through opening 25 and a lower, central protruding portion 26 with a number of protruding, flexible members 27 arranged to cooperate with said inner annular flange 16 on the abutment sleeve 2. Cap 22 comprises a lower, cylindrical sleeve-shaped portion 28 arranged to be inserted into the annular spacing formed between the flexible members 27 and the protruding screw head 4 of the coupling shaft 3. Cap 22 provides a closure for the through hole in the abutment sleeve 2 as well as a locking member for snap-in platform 24. Healing cap 37 is preferably made of a plastic material.

FIGS. 6-7 depict the sleeve-shaped portion 28 having a cylindrical shape. It is understood, however, that the sleeve-shaped portion 28 may have any other shape, such as a squared, rectangular, rounded or polygonal shape, depending on the connecting parts.

The entire implant device - including fixture 1, abutment sleeve 2, abutment screw 3 and healing cap 37 (discussed below) - may be installed in a single session followed by a healing period, instead of the traditional multi-step procedure in which the fixture is separately installed and followed by a healing period before the remaining parts, the abutment sleeve, abutment screw and healing cap, are installed.

Further features and advantages of the present invention may be found in U.S. Provisional Application No. 60/951,169, entitled "Coupling Apparatus For a Bone Anchored Hearing Device," and filed July 20, 2007, and U.S. Provisional Application No. 60/951,163, entitled "Bone Anchor Fixture for a Medical Prosthesis," and filed July 20, 2007.

The invention described and claimed herein is not to be limited in scope by the specific preferred embodiments herein disclosed, since these embodiments are intended as illustrations, and not limitations, of several aspects of the invention. Any equivalent embodiments are intended to be within the scope of this invention. Indeed, various modifications of the invention in addition to those shown and described herein will become apparent to those skilled in the art from the foregoing description. The scope of the invention is defined by the appended claims.

## Claims

1. A coupling apparatus (30) for anchorage of a prosthesis to a patient's skull bone, comprising:
an elongate anchoring fixture (1) comprising an external screw thread (6) for penetrating the bone, and a flange (7) that abuts the bone when the fixture (1) is implanted, the flange (7) comprising a flared external top portion (13) having a flared outer top surface; and
a skin-penetrating abutment sleeve (2) detachably connectable to said fixture (1) comprising a substantially conical exterior surface having a tapered outer surface having a tapered exterior contour (20) adjacent to the flared external top portion (13) of the flange (7),
**characterized in that**
a proximate end (1A) of the fixture (1) has an open cavity (14A) with a tapered interior contour (14); and
the tapered interior contour (14) of the elongate anchoring fixture (1) has a first cone angle (33), and the tapered exterior contour (20) of the skin-penetrating abutment sleeve (2) has a second cone angle (32) that is substantially the same as the first cone angle (33), so that the tapered exterior contour (20) adjacent to the flared external top portion (13) of the flange (7) fits within the tapered interior contour (14) of the open cavity (14A).

2. The coupling apparatus (30) of claim 1, wherein said prosthesis is a hearing prosthesis.

3. The coupling apparatus (30) of claim 1, wherein the flange (7) has a width that is greater than a peak diameter of the external screw threads (6).

4. The coupling apparatus (30) of claim 1, wherein the flange (7) comprises a cylindrical portion (12) adjacent to the flared external top portion (13).

5. The coupling apparatus (30) of claim 1, wherein the abutment sleeve (2) further comprises an upper end (17A) and an inner annular flange (16) disposed within the upper end (17A).

6. The coupling apparatus (30) of claim 1, wherein the coupling apparatus (30) further comprises an elongate coupling shaft (3) to securely couple the elongate anchoring fixture (1) and the skin-penetrating abutment sleeve (2).

7. The coupling apparatus (30) of claim 6, wherein the elongate coupling shaft (3) comprises a screw head (4).

8. The coupling apparatus (30) of claim 7, wherein the screw head (4) comprises an internal hex or multi-lobular configuration for a cooperating insertion tool.

9. The coupling apparatus (30) of claim 8, wherein the screw head (4) has an external hex geometry (29A) and a bore with an internal screw thread (30A).

10. The coupling apparatus (30) of claim 8, wherein the screw head (4) has an external thread (29B) and a bore with an internal unigrip configuration (30B).

11. The coupling apparatus (30) of claim 7, wherein the abutment sleeve (2) further comprises an internal shoulder (18) having a central opening for the elongate coupling shaft (3).

12. The coupling apparatus (30) according to any one of the claims 4 -11, wherein:
the flange (7) has a bottom portion (10) configured to prevent the bone anchor fixture (1) from penetrating through the bone; and
the flared outer top surface (13) of the bone anchor fixture (1) and the tapered outer surface (20) of the abutment sleeve (2) define an outer contour having a substantially hourglass shape having a waist and a waist angle (38).

13. The coupling apparatus (30) of claim 12, wherein the outer contour is substantially smooth.

14. The coupling apparatus (30) of claim 12, wherein the waist angle (38) is greater than 90°.

15. The coupling apparatus (30) of claim 12, wherein the abutment sleeve (2) is coated with a material to reduce the shear modulus.

16. The coupling apparatus (30) of claim 12, wherein the abutment sleeve (2) is coated with a surface increasing material.

17. The coupling apparatus (30) according to any one of the claims 1 - 16, further comprising a healing cap (37) comprising
a platform (24) comprising a central opening (25) and one or more protruding members (26, 27) configured to removably attach the healing cap (37) to coupling apparatus (30);
a cap (22) for the central opening (25); and
a flexible band (23) connecting the platform (24) and the cap (22).

18. The coupling apparatus (30) according to claim 17, wherein the one or more protruding members (26, 27) are provided around the central opening (25) to define an annular space.

19. The coupling apparatus (30) according to claim 18,
wherein the cap (22) further comprises a lower, cylindrical sleeve-shaped portion (28) configured to be inserted into the annular space.

20. The coupling apparatus (30) according to any one of the claims 1 - 19, further comprising:
coupling means (3) for securely and removably coupling the bone anchor means (1) and the abutment means (2),
wherein the bone anchor means (1) and the abutment means (2) define an outer contour having a substantially hourglass shape having a waist and a waist angle (38).

## Patentansprüche

1. Kopplungsvorrichtung (30) zum Verankern einer Prothese an einem Schädelknochen eines Patienten, umfassend:
eine langgestreckte Verankerungshalterung (1), die ein äußeres Schraubengewinde (6) zum Eindringen in den Knochen und einen Flansch (7) umfasst, der am Knochen anliegt, wenn die Halterung (1) implantiert ist, wobei der Flansch einen gebördelten externen oberen Bereich (13) mit einer gebördelten äußeren oberen Fläche umfasst; und
eine hautdurchdringende Auflagerhülse (2), die lösbar mit der Halterung (1) verbindbar ist, die eine im Wesentlichen konische Außenfläche mit einer kegelförmigen äußeren Fläche mit einer kegelförmigen Außenkontur (20) neben dem gebördelten externen oberen Bereich (13) des Flansches (7) umfasst,
**dadurch gekennzeichnet, dass**
ein benachbartes Ende (1A) der Halterung (1) einen offenen Hohlraum (14A) mit einer kegelförmigen Innenkontur (14) aufweist; und
die kegelförmige Innenkontur (14) der langgestreckten Verankerungshalterung (1) einen ersten Kegelwinkel (33) aufweist, und die kegelförmigen Außenkontur (20) der hautdurchdringende Auflagerhülse (2) einen zweiten Kegelwinkel (32) aufweist, der im Wesentlichen der selbe ist wie der erste Kegelwinkel (33), so dass die kegelförmige Außenkontur (20) neben dem gebördelten externen oberen Bereich (13) des Flansches (7) in die kegelförmige Innenkontur (14) des offenen Hohlraums (14A) passt.

2. Kopplungsvorrichtung (30) nach Anspruch 1, wobei die Prothese eine Gehörprothese ist.

3. Kopplungsvorrichtung (30) nach Anspruch 1, wobei der Flansch (7) eine Breite aufweist, die größer als der Maximaldurchmesser des äußeren Schraubengewindes (6) die Prothese eine Gehörprothese ist.

4. Kopplungsvorrichtung (30) nach Anspruch 1, wobei der Flansch (7) einen zylindrischen Bereich(12) neben dem gebördelten externen oberen Bereich (13) umfasst.

5. Kopplungsvorrichtung (30) nach Anspruch 1, wobei die Auflagerhülse (2) weiterhin ein oberes Ende (17A) und einen inneren kranzförmigen Flansch (16), der in dem oberen Ende (17A) angeordnet ist, umfasst.

6. Kopplungsvorrichtung (30) nach Anspruch 1, wobei die Kopplungsvorrichtung (30) weiterhin einen langgestreckten Kopplungsschaft (3) umfasst, um die langgestreckte Verankerungshalterung (1) und die hautdurchdringende Auflagerhülse (2) sicher zu koppeln.

7. Kopplungsvorrichtung (30) nach Anspruch 6, wobei der langgestreckte Kopplungsschaft (3) einen Schraubkopf (4) umfasst.

8. Kopplungsvorrichtung (30) nach Anspruch 7, wobei der Schraubkopf (4) eine Innensechskant- oder eine Innenvielzahn-Konfiguration für ein dazu passendes Einsatzwerkzeug umfasst.

9. Kopplungsvorrichtung (30) nach Anspruch 8, wobei der Schraubkopf (4) eine Außensechskantgeometrie (29A) und ein Loch mit einem internen Schraubengewinde (30A) aufweist.

10. Kopplungsvorrichtung (30) nach Anspruch 8, wobei der Schraubkopf (4) ein externes Gewinde (29B) und ein Loch mit einer internen Unigrip-Konfiguration (30B) aufweist.

11. Kopplungsvorrichtung (30) nach Anspruch 7, wobei die Auflagerhülse (2) weiterhin eine Innenschulter (18) mit einer zentralen Öffnung für den langgestreckten Kopplungsschaft (3) umfasst.

12. Kopplungsvorrichtung (30) nach einem der Ansprüche 4 - 11, wobei:
der Flansch (7) einen unteren Bereich (10) aufweist, der konfiguriert ist, zu verhindern, dass die Knochenverankerungshalterung (1) durch den Knochen durchgeht; und
die gebördelte äußere obere Fläche (13) der Knochenverankerungshalterung (1) und die kegelförmige Außenfläche (20) der Auflagerhülse (2) eine äußere Kontur mit einer im wesentlichen Sanduhrform mit einer Taille und einem Taillenwinkel (38) definieren.

13. Kopplungsvorrichtung (30) nach Anspruch 12, wobei die äußere Kontur im Wesentlichen glatt ist.

14. Kopplungsvorrichtung (30) nach Anspruch 13, wobei der Taillenwinkel (38) größer als 90° ist.

15. Kopplungsvorrichtung (30) nach Anspruch 12, wobei die Auflagerhülse (2) mit einem Material beschichtet ist, um das Schermodul zu verringern.

16. Kopplungsvorrichtung (30) nach Anspruch 12, wobei die Auflagerhülse (2) mit einem oberflächenvergrößerndem Material beschichtet ist.

17. Kopplungsvorrichtung (30) nach einem der Ansprüche 1 - 16, das weiterhin eine Heilkappe (37) umfasst, die Folgendes umfasst:
eine Plattform (24), die eine zentrale Öffnung (25) und ein oder mehrere vorspringende Elemente (26, 27), die konfiguriert sind, die Heilkappe (37) lösbar an die Kopplungsvorrichtung (30) anzubringen, umfasst;
einen Deckel (22) für die zentrale Öffnung(25); und
ein flexibles Band (26, 27), das die Plattform (24) und den Deckel (22) verbindet.

18. Kopplungsvorrichtung (30) nach Anspruch 17, wobei die ein oder mehreren vorspringenden Elemente (26, 27) um die zentrale Öffnung (25) bereitgestellt sind, um einen ringförmigen Raum zu definieren.

19. Kopplungsvorrichtung (30) nach Anspruch 18, wobei der Deckel (22) weiterhin einen unteren zylindrischen hülsenförmigen Bereich (28) umfasst, der konfiguriert ist, in den ringförmigen Raum eingesetzt zu werden.

20. Kopplungsvorrichtung (30) nach einem der Ansprüche 1 - 19, das weiterhin umfasst:
eine Kopplungseinrichtung (3) um die Knochenverankerungseinrichtung (1) und die Auflagereinrichtung (2) sicher und lösbar zu koppeln,
wobei die Knochenverankerungseinrichtung (1) und die Auflagereinrichtung (2) eine äußere Kontur mit einer im wesentlichen Sanduhrform mit einer Taille und einem Taillenwinkel definieren.

## Revendications

1. Dispositif d'accouplement (30) pour l'ancrage d'une prothèse sur l'os crânien d'un patient, comprenant :
une fixation de dispositif d'ancrage allongée (1) comprenant un filet externe (6) destiné à pénétrer dans l'os et une bride (7) qui vient en butée sur l'os lorsque la fixation (1) est implantée, la bride (7) comprenant une partie supérieure externe évasée (13) ayant une surface supérieure externe évasée ; et
un manchon de butée pénétrant dans la peau (2) pouvant être relié de manière amovible à ladite fixation (1) comprenant une surface extérieure sensiblement conique ayant une surface externe conique possédant un contour extérieur conique (20) adjacent à la partie supérieure externe évasée (13) de la bride (7),
**caractérisé en ce que**
l'extrémité proximale (1A) de la fixation (1) comporte une cavité ouverte (14A) avec un contour intérieur conique (14) ; et
le contour intérieur conique (14) de la fixation de dispositif d'ancrage allongée (1) possède un premier angle de cône (33) et le contour extérieur conique (20) du manchon de butée pénétrant dans la peau (2) possède un second angle de cône (32) qui est sensiblement égal au premier angle de cône (33), de sorte que le contour extérieur conique (20) adjacent à la partie supérieure externe évasée (13) de la bride (7) s'ajuste à l'intérieur du contour intérieur conique (14) de la cavité ouverte (14A).

2. Dispositif d'accouplement (30) selon la revendication 1, dans lequel ladite prothèse est une prothèse auditive.

3. Dispositif d'accouplement (30) selon la revendication 1, dans lequel la bride (7) possède une largeur supérieure au diamètre de pointe des filets externes (6).

4. Dispositif d'accouplement (30) selon la revendication 1, dans lequel la bride (7) comprend une partie cylindrique (12) adjacente à la partie supérieure externe évasée (13).

5. Dispositif d'accouplement (30) selon la revendication 1, dans lequel le manchon de butée (2) comprend en outre une extrémité supérieure (17A) et une bride annulaire interne (16) disposée à l'intérieur de l'extrémité supérieure (17A).

6. Dispositif d'accouplement (30) selon la revendication 1, dans lequel le dispositif d'accouplement (30) comprend en outre un arbre de couplage allongé (3) destiné à coupler fermement la fixation de dispositif d'ancrage allongée (1) et le manchon de butée pénétrant dans la peau (2).

7. Dispositif d'accouplement (30) selon la revendication 6, dans lequel l'arbre de couplage allongé (3) comprend une tête de vis (4).

8. Dispositif d'accouplement (30) selon la revendication 7, dans lequel la tête de vis (4) comprend une configuration hexagonale interne ou à lobes multiples pour un outil d'insertion coopérant.

9. Dispositif d'accouplement (30) selon la revendication 8, dans lequel la tête de vis (4) possède une géométrie hexagonale externe (29A) et un alésage avec un taraudage intérieur (30A).

10. Dispositif d'accouplement (30) selon la revendication 8, dans lequel la tête de vis (4) possède un filet externe (29B) et un alésage avec une configuration interne unigrip (30B).

11. Dispositif d'accouplement (30) selon la revendication 7, dans lequel le manchon de butée (2) comprend en outre un épaulement interne (18) comportant une ouverture centrale pour l'arbre de couplage allongé (3).

12. Dispositif d'accouplement (30) selon l'une quelconque des revendications 4 à 11, dans lequel :
la bride (7) possède une partie inférieure (10) configurée pour empêcher la fixation de dispositif d'ancrage sur l'os (1) de pénétrer dans l'os ; et
la surface supérieure externe évasée (13) de la fixation de dispositif d'ancrage sur l'os (1) et la surface externe conique (20) du manchon de butée (2) définissent un contour externe ayant sensiblement la forme d'un sablier possédant une taille et un angle de taille (38).

13. Dispositif d'accouplement (30) selon la revendication 12, dans lequel le contour externe est sensiblement lisse.

14. Dispositif d'accouplement (30) selon la revendication 12, dans lequel l'angle de taille (38) est supérieur à 90°.

15. Dispositif d'accouplement (30) selon la revendication 12, dans lequel le manchon de butée (2) est recouvert d'un matériau permettant de diminuer le module de cisaillement.

16. Dispositif d'accouplement (30) selon la revendication 12, dans lequel le manchon de butée (2) est recouvert d'un matériau accroissant la surface.

17. Dispositif d'accouplement (30) selon l'une quelconque des revendications 1 à 16, comprenant en outre un capuchon de cicatrisation (37) comprenant
une plate-forme (24) comprenant une ouverture centrale (25) et un ou plusieurs éléments en saillie (26, 27) configurés pour fixer de manière amovible le capuchon de cicatrisation (37) au dispositif d'accouplement (30) ;
un capuchon (22) pour l'ouverture centrale (25) ; et
une bande souple (23) reliant la plate-forme (24) et le capuchon (22).

18. Dispositif d'accouplement (30) selon la revendication 17, dans lequel le ou les éléments en saillie (26, 27) sont prévus autour de l'ouverture centrale (25), définissant un espace annulaire.

19. Dispositif d'accouplement (30) selon la revendication 18, dans lequel le capuchon (22) comprend en outre une partie cylindrique inférieure en forme de manchon (28) configurée pour être insérée dans l'espace annulaire.

20. Dispositif d'accouplement (30) selon l'une quelconque des revendications 1 à 19, comprenant en outre :
un moyen de couplage (3) pour coupler fermement et de façon amovible le moyen d'ancrage sur l'os (1) et le moyen de butée (2),
dans lequel le moyen d'ancrage sur l'os (1) et le moyen de butée (2) définissent un contour externe ayant sensiblement la forme d'un sablier ayant une taille et un angle de taille (38).
